Europäisches Patentamt

(19) European Patent Office

Office européen des brevets

(11) Publication number: **0 178 007**
**B1**

(12) **EUROPEAN PATENT SPECIFICATION**

(45) Date of publication of patent specification: **27.06.90**

(51) Int. Cl.⁵: **C 01 B 3/36**

(21) Application number: **85201545.2**

(22) Date of filing: **25.09.85**

(54) Process for the production of synthesis gas.

(30) Priority: **10.10.84 GB 8425620**

(43) Date of publication of application:
**16.04.86 Bulletin 86/16**

(45) Publication of the grant of the patent:
**27.06.90 Bulletin 90/26**

(84) Designated Contracting States:
**AT BE DE FR IT NL SE**

(56) References cited:
**EP-A-0 142 887**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Jacometti, Jack
Carel van Bylandtlaan 30
NL-2596 HR The Hague (NL)**
Inventor: **van der Burgt, Maarten Johannes
Carel van Bylandtlaan 30
NL-2596 HR The Hague (NL)**
Inventor: **de Graaf, Johannes Didericus
Carel van Bylandtlaan 30
NL-2596 HR The Hague (NL)**

(74) Representative: **Aalbers, Onno et al
P.O. Box 302
NL-2501 CH The Hague (NL)**

Courier Press, Leamington Spa, England.

**Description**

The invention relates to a process for the production of synthesis gas with an increased $H_2$/CO-ratio from hydrocarbons comprising the following steps:

a) at least one gaseous hydrocarbon is converted into a synthesis gas comprising $H_2$ and CO by partial oxidation with an oxygen-containing gas;

b) at least one gaseous hydrocarbon is converted with steam into a gaseous mixture comprising $H_2$ and CO;

c) the gaseous mixture comprising $H_2$ and CO, formed in step (b) is separated into a CO-containing stream and a $H_2$ stream;

d) the $H_2$/CO-ratio of the synthesis gas produced in step (a) is increased by adding at least part of the $H_2$ stream obtained in step (c) thereto.

An object of the invention is to increase the $H_2$/CO-ratio of a synthesis gas resulting from the conversion of in particular normally gaseous hydrocarbons thereby enabling the reaction of the carbon compounds with an optimal or near optimal consumption of energy and materials in the manufacture of synthesis gas from gaseous fuels. Suitable by-products and waste products from chemical synthesis and natural gas can be employed as the gaseous fuels.

In the autothermic partial oxidation of hydrocarbons the necessary thermal energy is supplied by the process itself via partial combustion of the feedstock. This technique is also technically involved as well as requiring an oxygen-containing gas.

The oxygen-containing gas may be pure oxygen, air or mixtures of pure oxygen and air.

In view of the depletion of oil reserves, the partial oxidation of gaseous fuels to synthesis gas, which is a feedstock for many chemicals, is a subject of growing interest.

The partial oxidation of gaseous feedstocks can take place according to various established processes.

These processes include the Shell Gasification Process. A comprehensive survey of this process can be found in the Oil and Gas Journal, September 6, 1971, pp 85—90.

The partial oxidation of gaseous fuels is usually carried out at temperatures around 900 to roughly 1600°C, preferably 1100 to 1500°C and pressures up to 100 bar, preferably 5 to 100 bar.

As a consequence of the high partial oxidation temperature and the use of gaseous fuels as feedstocks, the resulting synthesis gas advantageously contains no ash, slag, soot or tar, thereby eliminating the necessity of using expensive purification steps. Raised pressure, high temperature and a gaseous feed lead to a high degree of conversion and relative to the volume of the gasification chamber, they effect a high specific throughput. In the majority of plants in which synthesis gas converted into products such as ammonia, Oxo compounds, methanol or products from the Fischer-Tropsch synthesis and which operate under pressure, a considerable part of the investment required for the compression can be saved on partial oxidizing gaseous fuels under pressure. Compared to the established gasification processes in which ash-containing fuel is employed or which operate under normal pressure, the pressure partial oxidation of gaseous fuel permits a considerable saving in the manufacturing costs of synthesis gas.

The endothermic conversion of gaseous hydrocarbons with steam is described in "Encyclopedia of chemical technology" by Kirk-Othmer (3rd edition, 1980); Volume 21, page 543.

Endotheric thermal or catalytic reaction of gaseous hydrocarbons, in the presence of steam to produce carbon monoxide and hydrogen is an established reaction which is operated industrially using various processes. Such a process can be carried out in a tubular reactor, the necessary heat of reaction being supplied via heat transport through the walls of the tubes or in a medium of externally heated solid heat exchanger material e.g. fine grained solid which is used in a fluidized bed.

The known processes necessitate involved technology and exhibit low thermal efficiency.

As mentioned hereinbefore the gaseous mixture comprising $H_2$ and CO formed in the conversion of normally gaseous hydrocarbon(s) with steam (step b) is separated into a CO-containing stream and an $H_2$ stream. This separation step (c) can be carried out in every possible way e.g. cryogenically, by means of membranes, adsorption and/or adsorption.

It is preferably carried out by means of a pressure swing adsorption process. Such a process is described in US patent specification No. 3,699,218. The CO-containing stream resulting from separation step (c) mainly consists of CO, $CO_2$ and $CH_4$. At least part of it is preferably used for burning and at least part of the heat generated in this way is used for the conversion of the normally gaseous hydrocarbons in step (b).

It will be noticed that especially combining said steps a, b and c is very advantageous to increase the $H_2$/CO-ratio in the process as mentioned above.

At least part of the substantially pure hydrogen stream separated in step (c) is mixed with the synthesis gas produced in step (a). In this manner the $H_2$/CO-ratio of this gas is preferably increased to a value in the range from 1.8 to 2.5. Such a gas is very suitable for the synthesis of hydrocarbons according to the Fischer-Tropsch process, which process is described in the book "The Fischer-Tropsch and Related Syntheses" by H. H. Storch, N. Golumbic and R. B. Anderson (1951). Therefore the gas obtained in step (d) is at least in part catalytically converted into a product comprising a hydrocarbon mixture. The invention also relates to a process for the production of a hydrocarbon mixture by the catalytic conversion of the synthesis gas produced in step (d) of the present synthesis gas generating process.

This catalytic conversion is preferably carried out as described in one or more of the following patent documents: UK patent specifications Nos. 1,548,468 and 2,077,289; UK patent applications Nos. 8,320,715; 8,330,993; 8,330,994 and 8,413,595; and Netherlands patent application No. 8,303,909.

The product comprising a hydrocarbon mixture is preferably separated into a gaseous stream comprising non-converted CO and $H_2$ and low-boiling hydrocarbons, and at least one stream comprising normally liquid hydrocarbons. This separation can be carried out in any possible manner. Advantageously fractional distillation is used for this purpose.

The low-boiling hydrocarbons present in the gaseous stream also comprising CO and $H_2$, suitably consist of $C_1$—$C_4$-hydrocarbons, but higher boiling hydrocarbons may be present in this stream too. At least part of the gaseous stream comprising CO and $H_2$ and low-boiling hydrocarbons is preferably burned, suitably in a furnace, and at least part of the heat generated by this burning is used for the endothermic conversion of at least part of the normally gaseous hydrocarbons with steam in step (b) of the present synthesis gas generating process.

According to another preferred embodiment of the present invention at least part of the gaseous stream comprising CO and $H_2$ and low-boiling hydrocarbons is used as at least a part of the feed to step (b) in order to be converted with steam into the gaseous mixture comprising $H_2$ and CO.

According to still another embodiment of the invention at least part of the gaseous stream comprising CO and $H_2$ and low-boiling hydrocarbons is recycled to step (a) to be partially oxidized to synthesis gas.

As mentioned hereinbefore at least one stream comprising normally liquid hydrocarbons is preferably separated from the product comprising a hydrocarbon mixture.

Preferably at least part of this (these) normally liquid hydrocarbons-containing stream(s) is (are) catalytically hydrocracked, advantageously using at least part of the $H_2$ stream separated in step (c) of the present process.

This hydrocracking step can be carried out in any possible way known in the art. Preference is given to a hydrocracking process using a supported noble metal catalyst as described in European patent application No. 84,201,256.9.

The product of the hydrocracking step is preferably subjected to a fractional distillation, at least one normally gaseous fraction comprising non-converted hydrogen and low-boiling hydrocarbons, at least one naphtha fraction, at least one kerosene fraction and at least one gas oil fraction being separated from the hydrocracking product. The naphtha, kerosene and gas oil fractions are withdrawn from the present process as final products.

The normally gaseous fraction(s) comprising non-converted hydrogen and low-boiling hydrocarbons (suitably $C_1$-$C_4$) is (are) preferably recycled as a feed to step (a) and/or step (b) of the present process and/or is (are) advantageously burned for generating heat which is at least in part used in step (b) of the present synthesis gas production process.

The synthesis gas with increased $H_2$/CO-ratio which is produced in step (d) of the present synthesis gas production process can not only be used for the production of hydrocarbons but also as a feedstock for methanol production.

Therefore at least part of the gas obtained in step (d) is advantageously converted into a product comprising methanol.

Suitably a gaseous stream comprising non-converted CO and $H_2$ is separated from this product and at least part thereof is burned for generating heat which is at least in part used for the conversion of at least part of the gaseous hydrocarbons with steam in step (b) of the present synthesis gas production process.

Substantially pure methanol is separated from the product of the methanol synthesis step and withdrawn as the final product of this step, while heavier products than methanol being obtained in this separation step are advantageously recycled to steps (a) or (b) of the present process to be used as feedstock for these steps and/or as a means for generating heat for step (b).

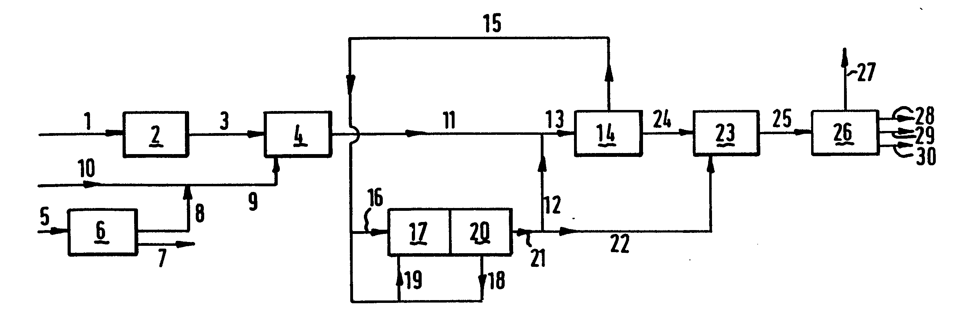

The present process will now be further elucidated by means of Figure 1 of the drawing, which represents a simplified flow scheme of this process, to which flow scheme the invention is by no means restricted.

Natural gas is introduced via a line 1 into a desulphurization unit 2 where sulphur compounds ($H_2S$) are removed therefrom. The desulphurized natural gas stream is passed via a line 3 into a gasification unit 4 where it is converted by partial oxidation into synthesis gas, mainly consisting of CO and $H_2$. To this end air is introduced via a line 5 into an air separation unit 6 where it is separated into a $N_2$-containing stream, withdrawn via a line 7 and a substantially pure oxygen stream passed via a line 8 to a line 9 where it is mixed with steam introduced into the system via a line 10.

The oxygen/steam mixture is passed via the line 9 into the gasification unit 4 where it is reacted with the desulphurized natural gas. The resulting synthesis gas having an $H_2$/CO-ratio below 1.7 is withdrawn from the gasifier 4 via a line 11. It is mixed with substantially pure hydrogen introduced via a line 12 and the mixture having an $H_2$/CO-ratio in the range from 1.8 to 2.5 is passed via a line 13 to a hydrocarbon synthesis unit 14 where it is at least in part converted to hydrocarbons. Non-converted $H_2$ and CO-containing gas mixture together with low boiling hydrocarbons ($C_1$-$C_4$) is withdrawn from the unit 14 via a line 15. Part of this gas mixture is introduced via a line 16 into a steam reforming unit 17 where it is converted with steam (not shown) into an $H_2$ and CO-containing gas mixture having a higher $H_2$/CO-ratio than the synthesis gas produced in the gasifier 4. Part of the gas mixture withdrawn from the unit 14 via the line 15 is mixed with a CO-containing stream introduced via a line 18 into a line 19 and the mixture is passed via the line 19 to the burners (not shown) of the steam reforming unit 17 where the mixture is burned in order to furnish the heat for the endothermic steam reforming reactions.

The synthesis gas produced in unit 17 is passed to a pressure swing adsorber 20 in which it is separated into a CO-containing stream withdrawn via the line 18 and a $H_2$ stream withdrawn via a line 21. Part of the $H_2$ stream is passed via the line 12 to the line 13. Another part of the $H_2$ stream is passed via a line 22 to a hydrocracking unit 23 where it is used for hydrocracking normally liquid hydrocarbons produced in the synthesis unit 14 and passed from unit 14 to unit 23 through a line 24.

The hydrocracking product is passed from the hydrocracking unit 23 via a line 25 to a fractional distillation unit 26 where it is separated into a normally gaseous stream containing non-converted $H_2$ and light hydrocarbons, a naphtha stream, a kerosene stream and a gas oil stream, which streams are withdrawn from the system via lines 27, 28, 29 and 30 respectively.

## Claims

1. A process for the production of synthesis gas with an increased $H_2$/CO-ratio from hydrocarbons characterized in that:

a) at least one gaseous hydrocarbon is converted into a synthesis gas comprising $H_2$ and CO by partial oxidation with an oxygen-containing gas;

b) at least one gaseous hydrocarbon is converted with steam into a gaseous mixture comprising $H_2$ and CO;

c) the gaseous mixture comprising $H_2$ and CO, formed in step (b) is separated into a CO-containing stream and a $H_2$ stream; and

d) the $H_2$/CO-ratio of the synthesis gas produced in step (a) is increased by adding at least part of the $H_2$ stream obtained in step (c) thereto.

2. A process as claimed in claim 1, in which at least part of the CO-containing stream separated in step (c) is burned and at least part of the heat generated by this burning is used for the conversion of the normally gaseous hydrocarbons with steam in step (b).

3. A process as claimed in any one of the preceding claims, in which the $H_2$/CO-ratio of the synthesis gas is increased in step (d) to a value in the range from 1.8 to 2.5.

4. A process as claimed in any one of the preceding claims, in which the separation of step (c) is carried out by means of a pressure swing adsorption process.

5. A process as claimed in any one of the preceding claims, in which the synthesis gas with increased $H_2$/CO-ratio is at least in part catalytically converted into a product comprising a hydrocarbon mixture.

6. A process as claimed in claim 5 in which the product comprising a hydrocarbon mixture is separated into a gaseous stream comprising non-converted CO and $H_2$ and low-boiling hydrocarbons, and at least one stream comprising normally liquid hydrocarbons.

7. A process as claimed in claim 6 preceding claims in which at least part of the gaseous stream comprising CO and $H_2$ and low-boiling hydrocarbons is burned and at least part of the heat generated by this burning is used for the conversion of at least part of the normally gaseous hydrocarbons with steam in step (b).

8. A process as claimed in claim 6 or 7 in which at least part of the gaseous stream comprising CO and $H_2$ and low boiling hydrocarbons is recycled as at least part of the feed to step (b).

9. A process as claimed in any one of the claims 5—8, in which at least one stream comprising normally liquid hydrocarbons separated from the product comprising a hydrocarbon mixture is catalytically hydrocracked using at least part of the $H_2$ stream separated in step (c).

10. A process as claimed in claim 9, in which the hydrocracking product is subjected to fractional distillation and at least one normally gaseous fraction, at least one naphtha fraction, at least one kerosene fraction and at least one gas oil fraction are separated from this product.

11. A process as claimed in claim 10 in which the normally gaseous fraction(s) is (are) recycled as a feed to step (a) and/or step (b), and/or is (are) burned for generating heat which is at least in part used in step (b).

12. A process as claimed in any one of the preceding claims 1—4, in which the synthesis gas with increased $H_2$/CO-ratio is at least in part converted into a product comprising methanol.

13. A process as claimed in claim 12 in which the product comprising methanol is separated into a normally liquid product comprising methanol and a gaseous stream comprising non-converted CO and $H_2$.

14. A process as claimed in claim 13 in which at least part of the gaseous stream comprising non-converted CO and $H_2$ is burned and at least part of the heat generated by this burning is used for the conversion of at least part of the normally gaseous hydrocarbons with steam in step (b).

## Patentansprüche

1. Verfahren zur Erzeugung von Synthesegas mit erhöhtem $H_2$/CO-Verhältnis aus Kohlenwasserstoffen, dadurch gekennzeichnet, daß:

a) wenigstens ein gasförmiger Kohlenwasserstoff durch partielle Oxidation mit einem Sauerstoff-enthaltenden Gas zu einem Wasserstoff und Kohlenmonoxid umfassenden Synthesegas umgewandelt wird;

b) wenigstens ein gasförmiger Kohlenwasserstoff mit Dampf zu einem Wasserstoff und Kohlenmonoxid umfassenden gasförmigen Gemisch umgewandelt wird;

c) das in Stufe (b) gebildete, Wasserstoff und Kohlenmonoxid umfassende Gasgemisch in einen Kohlenmonoxid-enthaltenden Strom und einen Wasserstoffstrom aufgetrennt wird; und

d) das $H_2$/CO-Verhältnis des in Stufe (a) erzeugten Synthesegases durch Zusetzen wenigstens eines Teiles des in Stufe (c) erhaltenen Wasserstoffstroms erhöht wird.

2. Verfahren nach Anspruch 1, worin wenigstens ein Teil des in Stufe (c) abgetrennten Kohlenmonoxid-enthaltenden Stroms verbrannt wird und wenigstens ein Teil der durch dieses Verbrennen gebildeten Wärme für die Umwandlung der normalerweise gasförmigen Kohlenwasserstoffe mit Dampf in der Stufe (b) verwendet wird.

3. Verfahren nach einem der vorstehenden Ansprüche, worin das $H_2$/CO-Verhältnis des Synthesegases in Stufe (d) auf einen Wert im Bereich von 1,8 bis 2,5 erhöht wird.

4. Verfahren nach einem der vorstehenden Ansprüche, worin die Auftrennung in Stufe (c) mittels eines Druckwechseladsorptionsverfahrens vorgenommen wird.

5. Verfahren nach einem der vorstehenden Ansprüche, worin das Synthesegas mit erhöhtem $H_2$/CO-Verhältnis wenigstens teilweise katalytisch in ein ein Kohlenwasserstoffgemisch umfassendes Produkt umgewandelt wird.

6. Verfahren nach Anspruch 5, worin das ein Kohlenwasserstoffgemisch umfassende Produkt in einen gasförmigen Strom, der nicht umgewandeltes Kohlenmonoxid und nicht umgewandelten Wasserstoff un niedrig siedende Kohlenwasserstoffe umfaßt, und in wenigstens einen, normalerweise flüssige Kohlenwasserstoffe umfassenden Strom aufgetrennt wird.

7. Verfahren nach Anspruch 6, worin wenigstens ein Teil des Kohlenmonoxid und Wasserstoff und niedrig siedende Kohlenwasserstoffe umfassenden gasförmigen Stroms verbrannt wird und wenigstens ein Teil der durch dieses Verbrennen gebildeten Wärme zur Umwandlung von wenigstens einem Teil der normalerweise gasförmigen Kohlenwasserstoffe mit Dampf in Stufe (b) verwendet wird.

8. Verfahren nach Anspruch 6 oder 7, worin wenigstens ein Teil des Kohlenmonoxid und Wasserstoff und niedrig siedende Kohlenwasserstoffe umfassenden gasförmigen Stroms als wenigstens ein Teil des Einsatzmaterials zur Stufe (b) zurückgeführt wird.

9. Verfahren nach einem der Ansprüche 5 bis 8, worin wenigstens ein normalerweise flüssige Kohlenwasserstoffe umfassender Strom, der von dem ein Kohlenwasserstoffgemisch umfassenden Produkt abgetrennt worden ist, unter Verwendung wenigstens eines Teiles des in Stufe (c) abgetrennt Wasserstoffstroms einem katalytischen Hydrocracken unterworfen wird.

10. Verfahren nach Anspruch 9, worin das Hydrocrackprodukt einer fraktionierten Destillation unterworfen wird und wenigstens eine normalerweise gasförmige Fraktion, wenigstens eine Naphthafraktion, wenigstens eine Kerosinfraktion und wenigstens eine Gasölfraktion aus diesem Produkt abgetrennt werden.

11. Verfahren nach Anspruch 10, worin die normalerweise gasförmige Fraktion bzw. die normalerweise gasförmigen Fraktionen als Einsatzmaterial zur Stufe (a) und/oder zur Stufe (b) zurückgeführt wird bzw. werden und/oder zur Bildung von Wärme verbrannt wird bzw. werden, die zumindest teilweise in Stufe (b) ausgenützt wird.

12. Verfahren nach einem der vorstehenden Ansprüche 1 bis 4, worin das Synthesegas mit erhöhtem $H_2$/CO-Verhältnis zumindest teilweise in ein Methanol umfassendes Produkt umgewandelt wird.

13. Verfahren nach Anspruch 12, worin das Methanol umfassende Produkt zu einem normalerweise flüssigen, Methanol umfassenden Produkt und zu einem gasförmigen, nicht umgewandeltes Kohlenmonoxid und nicht umgewandelten Wasserstoff umfassenden Strom aufgetrennt wird.

14. Verfahren nach Anspruch 13, worin wenigstens ein Teil des gasförmigen, nicht umgewandeltes Kohlenmonoxid und nicht umgewandelten Wasserstoff umfassenden Stromes verbrannt wird und wenigstens ein Teil der durch dieses Verbrennen gebildeten Wärme zur Umwandlung wenigstens eines Teiles der normalerweise gasförmigen Kohlenwasserstoffe mit Dampf in Stufe (b) verwendet wird.

**Revendications**

1. Un procédé pour la production de gaz de synthèse présentant un rapport accru $H_2$/CO à partir d'hydrocarbures, caractérisé en ce que:

a) au moins un hydrocarbure gazeux est converti en un gaz de synthèse comportant du $H_2$ et du CO par oxydation partielle au moyen d'un gaz renfermant de l'oxygène;

b) au moins un hydrocarbure gazeux est converti par de la vapeur en un mélange gazeux comportant $H_2$ et CO;

c) le mélange gazeux comportant $H_2$ et CO, formé au stage (b) est séparé en un courant renfermant CO et en un courant de $H_2$; et

d) le rapport $H_2$/CO du gaz de synthèse produit au stade (a) est accru en y ajoutant au moins une partie du courant de $H_2$ obtenu au stade (c).

2. Un procédé tel que revendiqué dans la revendication 1, dans lequel au moins une partie du courant renfermant le CO, séparé au stade (c) est brulé et au moins une partie de la chaleur produite par cette combustion est utilisée pour la conversion des hydrocarbures habituellement gazeux avec la vapeur au stade (b).

3. Un procédé tel que revendiqué dans l'une quelconque des revendications précédentes, dans lequel le rapport $H_2$/CO du gaz de synthèse est accru dans le stade (d) jusqu'à une valeur dans la plage de 1,8 à 2,5.

4. Un procédé tel que revendiqué dans l'une quelconque des revendications précédentes, dans lequel la séparation du stade (c) est réalisée au moyen d'un procédé d'adsorption à pression oscillante.

5. Un procédé tel que revendiqué dans l'une quelconque des revendications précédentes, dans lequel le gaz de synthèse avec un rapport $H_2$/CO accru est au moins en partie catalytiquement converti en un produit constitué d'un mélange d'hydrocarbures.

6. Un procédé tel que revendiqué dans la revendication 5, dans lequel le produit comportant un mélange d'hydrocarbures est séparé en un courant gazeux comportant du CO et de l'$H_2$ non-convertis et des hydrocarbures à bas point d'ébullition, et au moins un courant comprenant habituellement des hydrocarbures liquides.

7. Un procédé tel que revendiqué dans la revendication 6 et les revendications précédentes, dans lequel au moins une partie du courant gazeux comportant CO et $H_2$ et des hydrocarbures à bas point d'ébullition est brulée et au moins une partie de la chaleur produite par cette combustion est utilisée pour la conversion d'au moins une partie des hydrocarbures habituellement gazeux avec la vapeur dans l'étape (b).

8. Un procédé tel que revendiqué dans la revendication 6 ou 7, dans lequel au moins une partie du courant gazeux comportant CO et $H_2$ et des hydrocarbures à bas point d'ébullition est recyclée comme au moins une partie de la charge au stade (b).

9. Un procédé tel que revendiqué dans l'une quelconque des revendications 5 à 8, dans lequel au moins un courant comportant habituellement des hydrocarbures liquides séparé du produit comportant un mélange d'hydrocarbures est catalytiquement hydrocraqué en utilisant au moins une partie du courant de $H_2$ séparé au stade (c).

10. Un procédé tel que revendiqué dans la revendication 9, dans lequel le produit d'hydrocraquage est soumis à une distillation fractionnée et au moins une fraction habituellement gazeuse, au moins une fraction de naphta, au moins une fraction de kérosène et au moins une fraction de gazole sont séparées de ce produit.

11. Un procédé tel que revendiqué dans la revendication 10, dans lequel la (les) fraction(s) habituellement gazeuse(s) est (sont) recyclée(s) sous la forme d'une charge au stade (a) et/ou au stade (b), et/ou est (sont) brulée(s) pour produire de la chaleur qui est au moins en partie utilisée au stade (b).

12. Un procédé tel que revendiqué dans l'une quelconque des revendications précédentes 1 à 4, dans lequel le gaz de synthèse avec un rapport accru $H_2$/CO est au moins en partie converti en un produit comprenant du méthanol.

13. Un procédé tel que revendiqué dans la revendication 12, dans lequel le produit comprenant du méthanol est séparé en un produit habituellement liquide comprenant du méthanol et en un courant gazeux comprenant du CO et du $H_2$ non convertis.

14. Un procédé tel que revendiqué dans la revendication 13, dans lequel au moins une partie du courant gazeux comprenant du CO et du $H_2$ non convertis est brulée et au moins une partie de la chaleur produite par cette combustion est utilisée pour la conversion d'au moins une partie des hydrocarbures habituellement gazeux avec la vapeur au stade (b).